Europäisches Patentamt

European Patent Office

Office européen des brevets

(11) Publication number: **0 004 606**
**B1**

(12) **EUROPEAN PATENT SPECIFICATION**

(45) Date of publication of patent specification: **10.03.82**

(51) Int. Cl.³: **C 07 C 148/00**

(21) Application number: **79100874.1**

(22) Date of filing: **22.03.79**

(54) Process for preparing aniline compounds.

(30) Priority: **06.04.78 US 894132**
**19.10.78 US 952812**

(43) Date of publication of application:
**17.10.79 Bulletin 79/21**

(45) Publication of the grant of the patent:
**10.03.82 Bulletin 82/10**

(84) Designated Contracting States:
**BE CH DE FR GB**

(56) References cited:
**DE - B - 1 193 047**
**FR - A - 2 387 215**
**US - A - 3 406 202**

**BULLETIN OF THE CHEMICAL SOCIETY OF**
**JAPAN, volume 44, October 1971, TOKYO (JP)**
**KAZUHIKO TANAKA: "Studies of**
**Thioiminocarbonates. I. The Formation and**
**Decomposition of O-Alkyl S-Aryl**
**Thioiminocarbonates in the Reaction of Aryl**
**Thiocyanates with Alcohols in the Presence of**
**the Cyanide Ion", pages 2815—2820**

(73) Proprietor: **ETHYL CORPORATION**
**330 South Fourth Street P.O. Box 2189**
**Richmond Virginia 23219 (US)**

(72) Inventor: **Walter, Thomas James**
**10686 Danbury Drive**
**Baton Rouge Louisiana 70809 (US)**

(74) Representative: **Berg, Wilhelm, Dr. et al,**
**Dr. Berg, Dipl.-Ing. Stapf, Dipl.-Ing. Schwabe, Dr.**
**Dr. Sandmair Patentanwälte Postfach 860245**
**D-8000 München 86 (DE)**

(56) References cited:
**SPRINGER VERLAG, 1977 BERLIN,**
**HEIDELBERG, NEW YORK**
**W. P. WEBER: "Phase Transfer Catalysis in**
**Organic Synthesis", pages 225, 233.**

# 0 004 606

## Process for preparing aniline compounds

This invention relates to preparation of thioalkyl aniline compounds which are useful as intermediates in the preparation of anthelmintic, antibacterial and fungicidal agents. Recently, a number of patents have described anthelmintics of the benzimidazole type. Examples are U.S. 3,956,499, U.S. 3,915,896, U.S. 3,095,991, U.S. 3,682,952, Dutch Application 75/06095, British Patent 1,424,773, etc. Despite the disclosures of these patents, the present method for the preparation of such intermediates useful in such applications have not been heretofore disclosed.

In the prior art is shown methods of preparing alkyl aryl sulfides by the reaction of aryl thiocyanates with alcohols in the presence of alkali metal cyanides. Thus, Tanaka ("Bulletin of the Chemical Society of Japan", 44, pp. 2815—2820 (1971)) reacted o-chlorophenyl thiocyanate and n-propanol in the presence of sodium cyanide and obtained o-chlorophenyl propyl sulfide in a yield of 29 percent. When the reaction was carried out in the presence of dimethyl sulfoxide, a yield of 50 percent was realized. Tanaka also studied the reaction of diaryl disulfides with the cyanide ion and with dimethyl sulfide to obtain aryl alkyl sulfides, e.g. phenyl methyl sulfide ("Bulletin of the Chemical Society of Japan", 45, pp. 536—539 (1971)).

The present invention is directed to an improved process for making a specific class of alkyl aryl viz., alkyl aminoaryl sulfides, or as designated herein thioalkyl aniline compounds, and especially thioalkyl nitroaniline products.

In one form the present invention comprises the production of thioalkyl aniline compounds by the reaction of a thiocyano aniline and an alcohol in the presence of an alkali metal cyanide and a dipolar aprotic solvent improved in that an alkyl halide reactant is also provided. Another form of the invention comprises the production of the desired thioalkyl aniline compound products by reacting a thiocyano aniline with an alcohol and an alkyl halide in the presence of an alkali metal cyanide, a water immiscible solvent, and a phase transfer catalyst. In a further improved variation of this form, the invention is further characterized in that the phase transfer catalyst is a polymer-bound phase transfer catalyst.

The thioalkyl aniline compounds produced by the process of this invention can employ an alkyl group having from 1 to 4 carbon atoms. Preferably, only carbon and hydrogen are present in the structure of the alkyl group. Accordingly, groups such as methyl, ethyl, propyl, isopropyl, butyl and isobutyl groups can be used in the thioalkyl aniline compounds of the present invention.

Additionally, the aromatic ring of such thioalkyl aniline compounds can have such substituents as halo, nitro, amino and similar functional groups substituted on the benzene ring nucleus in the 2, 3, 5 or 6 position, positions 1 and 4 being reserved for the amino and thio groups, respectively.

Therefore, the compounds produced by the process of this invention can be 4-thiomethyl-2-nitro-aniline, 4-thioethyl-2-nitroaniline, 4-thiopropyl-2-nitroaniline, 4-thiobutyl-2-nitroaniline, 4-thiopropyl-3-nitroaniline, 4-thioethyl-3-chloroaniline, 4-thiobutyl-5-nitroaniline, 4-thiomethyl-6-nitroaniline, 4-thioethyl-2-bromoaniline, and 4-thiopropyl-3-aminoaniline. Of course, as indicated hereinabove, other groups which are innocuous to the cyanide displacement can be substituted for the nitro group in the same or different position. Most preferred of the compounds produced by the present process is 4-thiopropyl-2-nitroaniline because of its use as an intermediate in the synthesis of effective anthelmintics.

The above compounds are prepared by reaction of 4-thiocyano aniline compounds which are known intermediates for antibacterial compounds with an alcohol or other source of alkyl groups suitable for the formation of compounds of this invention in the presence of an equivalent of an alkali metal cyanide.

In the present invention, the alcohol employed is one source of alkyl groups in the thioalkyl or aniline compounds produced by the present process. Thus, methanol, ethanol, n-propanol, isopropanol and n-butanol may be used. As another source of alkyl groups, are used the alkyl halides. For example, alkyl chloride and alkyl bromide, such as methyl chloride, ethyl bromide, n-propyl chloride, n-propyl bromide, n-propyl iodide, n-butyl chloride and n-butyl bromide are useful. Any of the halides can be employed so long as no substantially adverse reaction with starting materials, intermediates or product is evidenced; although propyl bromide and propyl chloride are preferred because of good results and low cost. Most efficacious has been the combination of alcohol and alkyl halide. Any of the foregoing alcohols and alkyl halides can be used in about any relative amount. However, best results have been achieved when about molar equivalent quantities are employed. Most preferably, there is employed n-propanol with n-propyl bromide. Most highly preferred is the use of propanol and propyl bromide in molar equivalent ratios.

It has also been found that a dipolar aprotic solvent is effective to facilitate dissolution of the alkali metal cyanide into the reaction mixture. Many such solvents are known and commercially available. As examples, there may be mentioned dimethyl sulfoxide, dimethyl formamide and hexamethyl phosphoramide. Any of these which does not interfere with the reaction and easily accomplishes dissolution of the alkali metal cyanide into the reaction mixture can be used.

Although dipolar aprotic solvents provide advantageous process results, they suffer from the disadvantages of being expensive, high boiling and water soluble which makes product recovery and solvent recycle difficult. Therefore, an alternative process of this invention includes a process for

2

preparing a thioalkyl aniline by reacting a thiocyano aniline with an alcohol and an alkyl halide in the presence of an aqueous alkali metal cyanide, a water-immiscible solvent and a phase transfer catalyst. Catalysts useful in the process of the present invention are, in general, any compound having the general formula $(R)_4MX$ which provides the reaction system the ion "$(R)_4M^\oplus$" where M is a Group V—A element of Periodic Chart of the Elements, Fisher Scientific Company, Chicago, Illinois, 1955 (nitrogen, phosphorus, arsenic, antimony, or bismuth); at least one R group is a lower alkyl group and the remaining groups are alkyl groups having from 2 to 20 carbon atoms, benzyl or phenyl groups or mono-lower alkyl substituted benzyl or phenyl groups; and wherein X is a chlorine, iodine or bromine atom or a hydroxy or bisulfate group. Preferred $(R)_4MX$ compounds are those wherein the total number of carbon atoms in the $(R)_4$ portion are from 10 to 30, preferably from 12 to 20, or from 12 to 16, especially those wherein the R groups are acyclic groups. Preferred compounds are the ammonium or phosphonium compounds. Suitable phenyl groups are unsubstituted or substituted with compatible substituents which do not react adversely in the system. Preferably, the substitution on the phenyl groups is limited to that containing only hydrogen and carbon atoms such as lower alkyl groups, having from 1 to 4 carbon atoms, particularly methyl, ethyl, propyl or butyl groups. Typical catalyst compounds include tributyl methyl ammonium chloride [13 carbon atoms in the $(R)_4$ portion], tributyl methyl phosphonium chloride, tributyl methyl ammonium bromide, tributyl methyl ammonium iodide, tributyl methyl phosphonium bromide, tributyl methyl ammonium hydroxide, tributyl ethyl ammonium iodide [14 carbon atoms in the $(R)_4$ portion], tributyl ethyl ammonium bromide, tributyl ethyl ammonium chloride, tributyl methyl arsenic chloride, tributyl methyl antimony chloride, tributyl methyl bismuth chloride, dodecyltrimethyl ammonium chloride, didecyldimethyl ammonium chloride and tetraphenyl ammonium chloride. Other suitable catalysts are tripropyl butyl ammonium chloride, tripropyl butyl ammonium bromide and tripropyl butyl ammonium iodide. Other suitable catalysts are tripropyl pentyl ammonium chloride, tripropyl pentyl ammonium bromide and tripropyl pentyl ammonium iodide. Other suitable catalysts are tripropyl hexyl ammonium chloride, tripropyl hexyl ammonium bromide, tripropyl phenyl ammonium chloride, dipropyl methyl ethyl ammonium chloride, dimethyl ethyl phenyl ammonium chloride, tripropyl phenyl ammonium iodide and tributyltetradecyl phosphonium bromide $(Bu_3C_{14}H_{29}PBr)$.

The phase transfer catalyst provides reaction at the interface between reactants in the aqueous phase and the organic phase. The organic phase may be provided in a water-immiscible solvent. For reactions of this type it has been found that a halohydrocarbon solvent provides good water immiscibility, substantial inertness to reactants and products and good stability at reaction temperatures. Preferred halohydrocarbon solvents have a boiling point greater than 20°C at atmospheric pressure to provide good agitation during reaction, such as by refluxing. Typical of such water-immiscible solvents are methylene chloride, chloroform, chlorobenzene, trichloroethylene and perchloroethylene. Most preferred is methylene chloride because of its availability and utility.

In another aspect of the process of this invention, it has been found that recovery of the catalyst, recycle to subsequent reactions and easily handled product solutions are advantageously obtained when the phase transfer catalyst is immobilized or bound to a solid substate, such as a polymer which is not dissolved in either the aqueous or organic phase. Such a most highly preferred catalyst while maintaining yields equivalent to soluble phase transfer catalysts, do not become inactivated when chemically attached to the polymer substrate, are not lost during separation of the phases and are easily recovered for clean-up and recycle. For convenience, such catalysts will be referred to as polymer-bound phase transfer catalysts. The polymer portion of polymer-bound phase transfer catalyst is the organophilic portion and provides a solid substrate upon which the hydrophilic active portion of the polymer-bound phase transfer catalyst is attached. Any polymer, including coplymers, ternary polymers and the like can be employed providing they have the porosity, handling ability, physical integrity during reaction to form the phase transfer catalyst and chemical properties. Preferably, it has been found that a chloromethylstyrene-divinyl benzene copolymer, commercially available from Bio-Rad Laboratories, Richmond, California, under the tradename "Chloromethyl Biobeads" provides a suitable substrate for the preparation of the polymer-bound phase transfer catalyst used in the process of this invention. The above mentioned copolymer is reacted with, among others, a tertiary amine to form the polymer-bound phase transfer catalyst. Such tertiary amines, as are suitable in the formation of the quaternary salt catalysts of formula $(R)_4MX$ described hereinabove, are useful for the preparation of the polymer-bound phase transfer catalyst. For example, tertiary alkyl amines having from 1 to 20 carbon atoms in one or more of the alkyl groups, or benzyl phenyl groups in the tertiary amine are useful. Preferably, each alkyl group has from 1 to 12 carbon atoms and, more preferably, from 1 to 4 carbon atoms in each alkyl group. Typical examples are trimethyl amine, triethyl amine, tripropyl amine and tributyl amine, all as hereinbefore mentioned with respect to the quaternary salt catalyst. Preferred examples of such amines are N,N-dimethyl-n-butyl amine and tri-n-butyl amine.

Accordingly, the polymer bound phase transfer catalyst is the reaction product of tetrahydrofuran swollen 1% vinyl benzene crosslinked chloromethyl styrene with a tertiary amine which has 2 to 3 alkyl groups, each having 1 to 20 carbon atoms with a remaining group, if any, being a benzyl or phenyl or mono-lower alkyl-substituted benzyl or phenyl group.

The polymer-bound phase transfer catalysts useful in this invention can be prepared according to the method of Regen, *J. Org. Chem.,* Vol. 42, No. 5, 1977, pp. 875—879.

The polymer-bound phase transfer catalyst used in the process of this invention was prepared by soaking the "Chloromethyl Biobeads" in tetrahydrofuran until swollen. Then, a 50 to 100 percent molar excess of the N,N-dimethyl-*n*-butylamine was added and the reaction mixture was refluxed for 12 hours. The solids were filtered off, washed and dried overnight. The polymer-bound phase transfer catalyst was then ready for use as shown in Example 10.

The amount of catalyst used is, in general, an effective amount to achieve at least some catalytic effect; however, in general, one does not use more catalyst than is necessary to achieve a reasonable reaction rate since such usually involves the monopolization of reactor volume, needless expense and the like. In general, the amount of catalyst used ranges from 0.1 to 10 mol percent based on the 4-thiocyano-2-nitroaniline reactant with from 1 to 5 percent being preferred. Preferred catalysts contain a total of from 12 to 16 carbon atoms total in the R groups, especially those in which all R groups are not the same, since several such catalysts have an excellent relationship between the solubilities thereof in the reaction system as well as in water wash systems useful to remove catalyst residue from the product. Other criteria considered in the selection of catalysts include initial cost and ease of preparation. Outstanding catalysts are tetrabutyl ammonium bromide and tributyl methyl ammonium chloride, since they are readily prepared by a comparatively easy process known conventionally to one skilled in the art.

In each type of reaction alternatively described hereinabove, an alkali metal cyanide is employed. Preferably, the alkali metal cyanide is sodium or potassium cyanide. In the reactions employing a dipolar aprotic solvent the alkali metal cyanide is used *per se*, but when a phase transfer catalyst is employed the alkali metal cyanide may be an aqueous alkali metal cyanide.

The process for preparing the intermediates can be best illustrated by reference to a specific example. Since 4-thiopropyl-2-nitroaniline is typical of such intermediates, it will be used to illustrate the process for preparation of such useful compounds in the following examples.

## Example 1

A 0.40 g portion of powdered sodium cyanide was dissolved in a solution of 2.93 g of *n*-propanol and 8 ml of dimethylsulfoxide. Then, to the reactor was added 1.56 g of 4-thiocyano-2-nitroaniline and the reaction mass was stirred at room temperature. Another 0.4 g of powdered sodium cyanide was added after 105 minutes and dissolved into the reaction mass. Then, 145 minutes after the start of the reaction, 5 ml of propyl bromide was added to the reaction mixture which became thick and formed a porous solid so that the agitator blade was prevented from stirring. An additional 10 ml of dimethyl sulfoxide solvent was added. Workup of the reaction mixture by adding methylene chloride and extracting three times with water, followed by drying over sodium sulfate, showed the yield to be 98.6% of desired 4-thiopropyl-2-nitroaniline as determined by vapor phase chromatograph with internal standard.

## Example 2

Following the procedure of Example 1, except that propyl chloride and dimethyl formamide are used in place of propyl bromide and dimethyl sulfoxide. There was added 0.62 g of propanol, 0.81 g of propyl chloride and 0.122 g of biphenyl as an internal standard in dimethyl formamide. A 0.26 g portion of solid sodium cyanide was added to the solution and then 1 g of 4-thiocyano-2-nitroaniline was added to the reaction mixture which was stirred at room temperature. After 45 minutes, a sample analyzed by vapor phase chromatography showed 58% yield of 4-thiopropyl-2-nitroaniline. A yield of 67% 4-thiopropyl-2-nitroaniline was obtained in a sample analyzed by vapor phase chromatography after 1 hour and 15 minutes. Then, an additional 1.21 g of propyl chloride was added to the reaction mixture to hasten reaction. One hour later, a 71% yield of 4-thiopropyl-2-nitroaniline was determined by vapor phase chromatography.

## Example 3

A 0.30 g portion of powdered sodium cyanide was dissolved in a solution of 1.85 g of *n*-propanol in 10 ml of dimethyl sulfoxide. A 0.119 g portion of biphenyl was added as an internal standard to follow the reaction progress. Then, 4-thiocyano-2-nitroaniline in the amount of 1 g was added and the reaction mixture was stirred at room temperature. After 15 minutes from completion of the addition, the yield of 4-thiopropyl-2-nitroaniline was 27%. After one hour the yield was 31%. Since product formation had nearly stopped, 3.78 g of *n*-propyl bromide was added to the reaction mixture. Forty-five minutes later the yield of 4-thiopropyl-2-nitroaniline was 75%.

## Example 4

A 1 g portion of 4-thiocyano-2-nitroaniline, 0.16 g of tetrabutyl ammonium bromide as a catalyst, 0.117 g of biphenyl, 1.86 g of *n*-propanol, and 3.79 g of *n*-propyl bromide were dissolved in 10 ml of refluxing methylene chloride. To the refluxing reaction mixture was then added a 1.56 g portion of sodium cyanide as 6.16 g of a 25% aqueous solution. The reaction mixture was refluxed using a 50°C oil bath and moderate agitation. The reaction mixture turns a burgundy red after one or two minutes.

The following time-yield relationship was observed by taking samples and analyzing by vapor phase chromatography.

| Time (Min.) | 4-Thiopropyl-2-nitroaniline (% Yield) |
|---|---|
| 30 | 59 |
| 105 | 85 |
| 150 | 92 |
| 215 | 93 |

The reaction was stopped and the layers separated without emulsions. The organic layer was dried and the solvent evaporated on a rotary evaporator to obtain the crude product.

## Example 5

The procedure of Example 4 was followed except that n-propyl bromide was replaced by 2.42 g of n-propyl chloride and 0.102 g of biphenyl were employed as the internal standard. After addition of all the reagents in the same amounts as Example 4, with the exceptions noted above, the reaction mixture forms an orange precipitate. After 1 hour and 15 minutes, the yield of product 4-thiopropyl-2-nitroaniline is 18%. After 160 minutes, the yield of 4-thiopropyl-2-nitroaniline is only 20%. A solid was separated from the reaction mixture and was not further characterized.

## Example 6

A 1 g portion of 4-thiocyano-2-nitroaniline, 0.16 g of tetrabutyl ammonium bromide, 1.865 g of n-propanol and 3.791 g of n-propyl bromide was dissolved in 10 ml of refluxing methylene chloride. Then, 0.114 g of biphenyl was added as an internal standard to follow the reaction via vapor phase chromatograph. A 6 g portion of 25.3% aqueous sodium cyanide (1.51 g of sodium cyanide) was added to the reaction mixture. The reaction mixture was refluxed on an oil bath at 50°C with moderate agitation. The yield of 4-thiopropyl-2-nitroaniline was followed during the course of the reaction and after 30 minutes the yield of 4-thiopropyl-2-nitroaniline was 68.4%. After 2 hours, the yield was 91.1%. The reaction was stopped after 3 hours and 10 minutes and the yield was 87.7%.

## Example 7

This example is similar to Example 6, except that 1 mole % of the catalyst tetrabutyl ammonium bromide based on the starting 4-thiocyano-2-nitroaniline is employed. A 1 g portion of 4-thiocyano-2-nitroaniline, 0.016 g of tetrabutyl ammonium bromide, 1.86 g of n-propanol, 0.114 g of biphenyl and 3.79 g of n-propyl bromide were dissolved in 10 ml of refluxing methylene chloride. Then, 6 g of 25.3% aqueous sodium cyanide (containing 1.51 g of sodium cyanide) was added to the reactor. The reaction mixture was refluxed using a 50°C oil bath and moderate agitation. The reaction progress was followed on the vapor phase chromatograph with the following results:

| Reaction Time After Addition (hrs.) | 4-Thiopropyl-2-nitroaniline (% Yield) |
|---|---|
| 0.5 | 7 |
| 1.0 | 19 |
| 2.0 | 40 |
| 3.0 | 54 |
| 5.0 | 72 |
| 5.75 | 61 |

Trouble was experienced with the vapor phase chromatograph columns. On changing the columns, the final yield of a sample of this experiment was 85% of 4-thiopropyl-2-nitroaniline.

## Example 8

This experiment is similar to Example 6, except that 1 mole % of methyl tributyl ammonium chloride catalyst based on 4-thiocyano-2-nitroaniline is employed. The reagents as in Example 7 were used except that 0.131 g of biphenyl was added as an internal standard and 0.012 g of the methyl tributyl ammonium chloride was employed instead of the tetrabutyl ammonium bromide. Following the same procedure as in Example 7, the following readings were made by the vapor phase chromatograph with time:

| Time (Hrs.) | 4-Thiopropyl-2-nitroaniline (% Yield) |
|---|---|
| 1 | 24 |
| 2 | 42 |
| 4 | 64 |
| 5 | 72 |
| 6 | 77 |

## Example 9

A 2 g portion of 4-thiocyano-2-nitroaniline, 0.16 g of a 75 wt % aqueous solution of methyl tributyl ammonium chloride (equivalent to 5 mol% based on the 4-thiocyano-2-nitroaniline), 3.72 $n$-propanol and 7.58 g of $n$-propyl bromide was dissolved in 20 ml of refluxing methylene chloride. A 12 g portion of 25.3 wt % aqueous sodium cyanide (3.04 g of sodium cyanide) was added. The reaction mixture was refluxed using 50°C oil bath and moderate agitation. The reaction was stopped after 4 hours and the phases were separated. High pressure liquid chromatographic (HPLC) analysis determined a yield of 97% of 4-thiopropyl-2-nitroaniline.

In a separate experiment under identical conditions, a 97% yield of 4-thiopropyl-2-nitroaniline was determined using the internal standard vapor phase chromatographic analysis method.

The product can be purified by column chromatography and recrystallized from toluene-hexane solvent mixture. The orange needles of 4-thiopropyl-2-nitroaniline melt at 36.5—38°C. Infrared and NMR analysis confirm the structure. Alternatively, the product can be purified so as to recycle the aqueous phase and recover unreacted $n$-propanol, $n$-propyl bromide and methylene chloride. This involves an initial phase separation followed by flash of the methylene chloride and unreacted $n$-propanol and $n$-propyl bromide.

## Example 10

A flask was charged with 1.0 g of 4-thiocyano-2-nitroaniline, 6.0 g of an aqueous solution containing 25% sodium cyanide, 10 ml of methylene chloride, 0.22 g of polymer-bound phase transfer catalyst, prepared as described hereinabove, containing 1.19 meq. of quaternary nitrogens per gram of catalyst, 1.85 g of $n$-propanol, 3.78 g of $n$-propyl bromide, and 0.105 g of biphenyl as an internal standard. The stirred reaction mixture was placed in a 50°C oil bath for 6 hours. The catalyst was filtered and washed with methylene chloride. The methylene chloride layer was separated from the aqueous layer and analyzed by gas chromatography. The yield of 4-thiopropyl-2-nitroaniline was 89%.

The process is not limited to a tetrabutyl ammonium bromide, methyl tributyl ammonium chloride or polymer-bound phase transfer catalyst. Any compound known for this type of activity can be used. Many phase transfer catalysts are effective, such as the ammonium or phosphonium salts known to skilled practitioners to be effective as phase transfer catalysts. Similarly, solvents other than methylene chloride can be used. Further, the reaction temperature can be varied. Additionally, reaction times can be decreased by running the above reaction in an appropriate pressure vessel at pressures greater than atmospheric pressure. Reaction times can also be increased or decreased, depending on catalyst concentration.

**Claims**

1. A process for the production of a thioalkyl aniline compound by reacting a thiocyano aniline and an alcohol in the presence of an alkali metal cyanide and a dipolar aprotic solvent characterized in that the process is carried out in the additional presence of an alkyl halide.

2. The process of Claim 1 further characterized in that the thioalkyl aniline is a 4-thioalkyl-2-nitro aniline and the thiocyano aniline is a 4-cyano-2-nitro aniline, the alcohol and the alkyl halide each contain the same number of from 1 to 4 carbon atoms, the alkali metal cyanide is sodium cyanide or potassium cyanide, and the dipolar aprotic solvent is dimethyl sulfoxide, dimethyl formamide or hexamethyl phosphoramide.

3. The process of Claim 2 further characterized in that the alcohol is n-propanol and the alkyl halide is n-propyl chloride or n-propyl bromide.

4. A process for the production of a thioalkyl aniline by the reaction of a thiocyano aniline and an alcohol in the presence of an aqueous alkali metal cyanide characterized in that the reaction is conducted in the presence of a water immiscible solvent, an alkyl halide and a phase transfer catalyst.

5. The process of Claim 4 further characterized in that the thioalkyl aniline compound is a 4-thioalkyl-2-nitro aniline and the 4-thiocyano aniline is a 4-cyano-2-nitro aniline, the alcohol and alkyl halide each contain the same number of from 1 to 4 carbon atoms, the aqueous alkali metal cyanide is sodium cyanide or potassium cyanide, the immiscible solvent is a halohydrocarbon having a boiling point greater than about 20°C. at atmospheric pressure and is substantially inert to the reactants and products, and the phase transfer catalyst is a compound having the general formula $(R)_4MX$ in which M is a member of the group selected from nitrogen, phosphorus, arsenic, antimony and bismuth, at least one R group is a lower alkyl group and the remaining R groups are alkyl groups having from 2 to 20 carbon atoms, benzyl or phenyl groups, or mono- lower alkyl-substituted benzyl or phenyl groups and X is a halogen selected from chlorine, bromine or iodine, or a hydroxy or bisulphate group.

6. The process of Claim 5 further characterized in that the alcohol is n-propanol and the alkyl halide is n-propyl chloride or n-propyl bromide, the water immiscible solvent is methylene chloride, and the phase transfer catalyst is tetra-n-butyl ammonium bromide, methyl tri-n-butyl ammonium bromide or methyl tri-n-butyl ammonium chloride.

7. The process of Claims 4, 5 or 6 further characterized in that the phase transfer catalyst is a polymer-bound phase transfer catalyst.

8. The process of Claim 7 further defined in that the polymer-bound phase transfer catalyst is the reaction product of tetrahydrofuran swollen, 1% divinylbenzene cross-linked chloromethyl styrene with a tertiary amine which has two to three alkyl groups each having 1 to 20 carbon atoms with the remaining group if any being a benzyl or phenyl or mono-lower alkyl-substituted benzyl or phenyl group.

9. The process of Claim 8 further characterized in that the tertiary amine is N,N-dimethyl-n-butyl amine or tri-n-butyl amine.

## Patentansprüche

1. Verfahren zur Herstellung einer Thioalkylanilinverbindung durch Umsetzen eines Thiocyanoanilins mit einem Alkohol in Gegenwart eines Alkalimetallcyanids und eines dipolaren aprotischen Lösungsmittels, dadurch gekennzeichnet, daß das Verfahren in der zusätzlichen Gegenwart eines Alkylhalogenids durchgeführt wird.

2. Verfahren nach Anspruch 1, dadurch gekennzeichnet, daß das Thioalkylanilin ein 4-Thioalkyl-2-nitroanilin und das Thiocyanoanilin ein 4-Cyano-2-nitroanilin sind, daß der Alkohol und das Alkylhalogenid jeweils die gleiche Anzahl von 1 bis 4 Kohlenstoffatomen aufweisen, daß das Alkalimetallcyanid Natriumcyanid oder Kaliumcyanid ist und daß das dipolare aprotische Lösungsmittel Dimethylsulfoxid, Dimethylformamid oder Hexamethylphosphorsäuretriamid ist.

3. Verfahren nach Anspruch 2, dadurch gekennzeichnet, daß der Alkohol n-Propanol und das Alkylhalogenid n-Propylchlorid oder n-Propylbromid sind.

4. Verfahren zur Herstellung eines Thioalkylanilins durch Umsetzen eines Thiocyanoanilins mit einem Alkohol in Gegenwart eines wäßrigen Alkalimetallcyanids, dadurch gekennzeichnet, daß die Umsetzung in Gegenwart eines mit Wasser nicht mischbaren Lösungsmittels, eines Alkylhalogenids und eines Phasentransferkatalysators durchgeführt wird.

5. Verfahren nach Anspruch 4, dadurch gekennzeichnet, daß die Thioalkylanilinverbindung ein 4-Thioalkyl-2-nitroanilin und das 4-Thiocyanoanilin ein 4-Cyano-2-nitroanilin sind, daß der Alkohol und das Alkylhalogenid jeweils die gleiche Anzahl von 1 bis 4 Kohlenstoffatomen aufweisen, daß das wäßrige Alkalimetallcyanid Natriumcyanid oder Kaliumcyanid ist, daß das nicht mischbare Lösungsmittel ein Halogenkohlenwasserstoff mit einem Siedepunkt von mehr als etwa 20°C bei Atmosphärendruck ist, der gegenüber den Reaktionsteilnehmern und Produkten im wesentlichen inert ist, und daß der Phasentransferkatalysator eine Verbindung der allgemeinen Formel $(R)_4$ MX ist, worin M ein Vertreter der Stickstoff, Phosphor, Arsen, Antimon und Wismut umfassenden Gruppe ist, mindestens eine Gruppe R eine niedrigmolekulare Alkylgruppe und die restlichen Gruppen R Alkylgruppen mit 2 bis etwa 20 Kohlenstoffatomen, Benzylgruppen oder Phenylgruppen oder mononiedrigalkyl-substituierte Benzylgruppen oder Phenylgruppen sind und X ein aus der Chlor, Brom oder Jod umfassenden Gruppe ausgewähltes Halogen oder eine Hydroxygruppe oder eine Bisulfatgruppe bedeutet.

6. Verfahren nach Anspruch 5, dadurch gekennzeichnet, daß der Alkohol n-Propanol und das Alkylhalogenid n-Propylchlorid oder n-Propylbromid sind, das mit Wasser nicht mischbare Lösungsmittel Methylenchlorid ist und der Phasentransferkatalysator Tetra-n-butylammoniumbromid, Methyl-tri-n-butylammoniumbromid oder Methyl-tri-n-butylammoniumchlorid ist.

7. Verfahren nach Anspruch 4, 5 oder 6, dadurch gekennzeichnet, daß der Phasentransferkatalysator ein polymer-gebundener Phasentransferkatalysator ist.

8. Verfahren nach Anspruch 7, dadurch gekennzeichnet, daß der polymer-gebundene Phasentransferkatalysator das Reaktionsprodukt aus mit Tetrahydrofuran gequollenem, mit 1% Divinylbenzol vernetztem Chlormethylstyrol und einem tertiären Amin ist, welches zwei bis drei Alkylgruppen mit jeweils 1 bis 20 Kohlenstoffatomen aufweist und die gegebenenfalls noch vorhandene restliche Gruppe eine Benzylgruppe oder Phenylgruppe oder eine mono-niedrigalkylsubstituierte Benzyl- oder Phenylgruppe ist.

9. Verfahren nach Anspruch 8, dadurch gekennzeichnet, daß das tertiäre Amin N,N-Dimethyl-n-butylamin oder Tri-n-butylamin ist.

## Revendications

1. Procédé de production d'un composé thioalkylique d'aniline par réaction d'une thiocyano-aniline et d'un alcool en présence d'un cyanure de métal alcalin et d'un solvant aprotique dipolaire, caractérisé en ce qu'il est mis en oeuvre en la présence additionnelle d'un halogénure d'alkyle.

2. Procédé suivant la revendication 1, caractérisé en outre en ce que la thio-alkylaniline est une 4-thio-alkyl-2-nitro-aniline et la thiocyano-aniline est une 4-cyano-2-nitro-aniline, l'alcool et l'halogénure d'alkyle contiennent chacun le même nombre de 1 à 4 atomes de carbone, le cyanure de métal alcalin est le cyanure de sodium ou le cyanure de potassium et le solvant aprotique dipolaire est le diméthylsulfoxyde, le diméthylformamide ou l'hexaméthylphosphoramide.

3. Procédé suivant la revendication 2, caractérisé en outre en ce que l'alcool est le n-propanol et l'halogénure d'alkyle est le chlorure de n-propyle ou le bromure de n-propyle.

4. Procédé de production d'une thio-alkylaniline par réaction d'une thiocyano-aniline et d'un alcool en présence d'un cyanure de métal alcalin aqueux, caractérisé en ce que la réaction est conduite en présence d'un solvant non miscible à l'eau, d'un halogénure d'alkyle et d'un catalyseur de transfert de phase.

5. Procédé suivant la revendication 4, caractérisé en outre en ce que le composé thio-alkylique d'aniline est une 4-thio-alkyl-2-nitro-aniline et la 4-thiocyanoaniline est une 4-cyano-2-nitro-aniline, l'alcool et l'halogénure d'alkyle contiennent chacun le même nombre de 1 à atomes de carbone, le cyanure de métal alcalin aqueux est le cyanure de sodium ou de potassium, le solvant non miscible est un hydrocarbure halogéné ayant un point d'ébullition supérieur à environ 20°C à la pression atmosphérique et est sensiblement inerte aux corps réactionnels et aux produits et le catalyseur de transfert de phase est un composé de formule générale $(R)_4MX$ dans laquelle M est un représentant du groupe choisi entre l'azote, le phosphore, l'arsenic, l'antimoine et le bismuth, au moins un groupe R est un groupe alkyle inférieur et les groupes R restants sont des groupes alkyle ayant 2 à environ 20 atomes de carbone, des groupes benzyle ou phényle ou des groupes benzyle ou phényle portant un substituant alkyle inférieur et X est un halogène choisi entre le chlore, le brome ou l'iode, ou un groupe hydroxy ou bisulfate.

6. Procédé suivant la revendication 5, caractérisé en outre en ce que l'alcool est le n-propanol et l'halogénure d'alkyle est le chlorure de n-propyle ou le bromure de n-propyle, le solvant non miscible à l'eau est le chlorure de méthylène, et le catalyseur de transfert de phase est le bromure de tétra-n-butylammonium, le bromure de méthyl-tri-n-butylammonium ou le chlorure de méthyl-tri-n-butylammonium.

7. Procédé suivant la revendication 4, 5 ou 6, caractérisé en outre en ce que le catalyseur de transfert de phase est un catalyseur de transfert de phase lié à un polymère.

8. Procédé suivant la revendication 7, caractérisé en outre en ce que le catalyseur de transfert de phase lié à un polymère est le produit de réaction de chlorométhylstyrène gonflé au tétrahydrofuranne, réticulé par 1% de divinylbenzène, avec une amine tertiaire qui porte deux ou trois groupes alkyle ayant chacun 1 à 20 atomes de carbone, le groupe restant éventuel étant un groupe benzyle ou phényle ou un groupe benzyle ou phényle portant un substituant inférieur.

9. Procédé suivant la revendication 8, caractérisé en outre en ce que l'amine tertiaire est la N,N-diméthyl-n-butylamine ou la tri-n-butylamine.